# EUROPEAN PATENT APPLICATION

(11) **EP 4 395 298 A1**
(43) Date of publication of application: **03.07.2024**
(21) Application number: 22919955.9
(22) Date of filing: 01.12.2022
(51) Int. Cl.: H04N 5/33, A61N 2/00, A61B 5/00

(54) **APPARATUS FOR TRANSMITTING VARIABLE PULSES**

(30) Priority: 12.01.2022 US 202263299002 P; 30.06.2022 US 202263357624 P
(71) Applicant: Venitas Research Center, Inc., Keelung City, Taiwan 203 (CN)
(72) Inventor: HO, Conway, Zhongshan (CN); JIN, Derek Hertz, Zhongshan (CN)
(74) Representative: Straus, Alexander
(86) International application number: PCT/CN2022/135868
(87) International publication number: WO 2023/134322

(57) **Abstract**

A method of modulating a brain activity of a mammal which is achieved by subjecting the mammal to variable repetitive Transcranial Magnetic Stimulation (rTMS) with pulse intervals between 66.00 milliseconds (ms) and 200.00 ms. The method can also be used to by administering variable electric stimulation to the brain with pulse intervals between 66 milliseconds (ms) and 200 ms. The method is useful in treating brain dysfunctions. The variable pulse intervals are generated without the use of EEG data, derived from EEG data or other biometric measures.

## Description

### Related Applications

The present application claims priority to provisional application No. 63/299,002, filed on January 12, 2022 and provisional application No. 63/357,624, filed on June 30, 2022, the content of which is incorporated herein by reference in its entirety.

### Field of the Invention

The present invention relates to Apparatus of modulating a mammal's brain activity with repetitive transcranial magnetic stimulation (rTMS) wherein the rTMS is administered with variable pulse intervals for a time sufficient to modulate said brain activity wherein an improvement in a physiological condition or a clinical condition is achieved. The rTMS pules intervals range from 66.00 milliseconds to (ms) to 200.00 ms. Additionally, the present invention relates to electric stimulation of the brain by administering variable electric pulse intervals for a time sufficient to modulate said brain activity wherein an improvement in a physiological condition or a clinical condition is achieved. Preferably, the variable electromagnetic pulse intervals are delivered in a random fashion. The pulse interval timing in both magnetic and electric stimulation is predetermined and not dependent upon or derived from EEG or other biometric data of the mammal. The present invention also includes electromagnetic stimulation devices that can supply electric or magnetic stimulation comprising variable pulse intervals and preferably random pulse intervals independent of a patient's EEG data or data derived from EEG data or other biometric measures.

### Background of the Invention

Repetitive transcranial magnetic stimulation (rTMS) have been used to treat many psychological and medical disorders such as major depressive disorder, Parkinson's disease, PTSD, Alzheimer's disease, autism spectrum disorder (ASD), schizophrenia, pain management and others. Recently, Jin and Phillips, in US Patent Publication 2009/0082690, have disclosed a treatment protocol using rTMS where the output of the magnetic field is adjusted based on a patient's EEG intrinsic frequency in an attempt to alter the patient's intrinsic EEG frequency. US Patent 9,308,385 uses a different approach to administer rTMS by using a frequency based on a biological metric or a harmonic of a biological metric.

Other treatment methods use various means of identifying an optimum frequency to treat patients. All of the prior art treatment methods involving electromagnetic stimulation of the brain use a patient's EEG data to determine the electromagnetic frequency to use in administering the electromagnetic stimulation.

Prior to the present invention, rTMS treatments have consisted of the delivery of a single frequency as the electromagnetic stimulation at a set intensity.

### Summary of the Invention

The present invention provides a novel rTMS delivery system that delivers rTMS or electric stimulation with variable pulse intervals, of which the probability of interval is determined by a given type of noise function such as Gaussian, pink, or white noise. The power/intensity of the electromagnetic stimulation can also be variable. Additionally, the electric and magnetic stimulation devices are programmed to deliver variable pulse intervals of magnetic or electric stimulation. The present invention provides huge advantages over the prior art that uses single frequency delivery of magnetic or electric stimulation. The prior art delivers a fixed frequency stimulation or requires: (a) a patient's EEG or ECG and (b) the analysis of the biometric data to obtain a cleaner pattern and then the subjective interpretation of the data.

Briefly, in accordance with the present invention, an apparatus method of modulating a brain activity of a mammal is achieved by subjecting the mammal to repetitive Transcranial Magnetic Stimulation (rTMS) with variable pulse intervals between 66.00 milliseconds (ms) and 200.00 ms. The apparatus can also be used to by administering variable electric stimulation to the brain. The apparatus is useful in treating brain dysfunctions, diseases, or physical conditions. The variable pulse intervals in both magnetic stimulation and electric stimulation are generated by a preprogrammed noise generator such as Gaussian, pink, or white noise, and truncated within the range matching the rhythms of the brain activities. It does not use EEG data, data derived from EEG or other biometrics such as heart rate, respiration rate and the like.

Physiological conditions and medical conditions that can be improved by modulating the brain activity according to the present invention are any conditions where abnormal brain activity contributes to a specific condition. Conditions that are treated include, but are not limited to, autism spectrum disorder (ASD), Alzheimer's Disease (AD), Post Traumatic Stress Disorder (PTSD), Traumatic Brain Injury (TBI), memory impairment, depression, pain, addiction, substance abuse disorder (SUD), Obsessive Compulsive disorders (OCD), anxiety, Parkinson's disease, hypertension, libido dysfunction, motor function abnormalities, small height in young children, stress, obesity, sleep disorders, eating disorders, concentration/focus abnormalities, speech abnormalities, intelligence deficits, cognition abnormalities, Attention Deficit Hyperactivity Disorders (ADHD), schizophrenia, coma, bipolar disorders, tinnitus, fibromyalgia, chronic Lyme disease, Rheumatoid Arthritis (RA), other autoimmune diseases, gout, diabetes, arthritis, trauma rehab, athletic performance, cognitive improvement, long COVID symptoms (and in particular loss of smell and/or taste) and stroke.

Of particular interest in practicing the present invention, a patient is subjected to repetitive transcranial magnetic stimulation (rTMS) with random variable pulse intervals for a time sufficient to modulate a brain activity in the patient where an improvement in a physiological condition or a clinical condition is achieved. The improvement is observed by clinical evaluation both the patient and the patient's healthcare professional. The power or intensity of the magnetic stimulation is generally under the patient's motor threshold and preferably from 50-90 percent (%) of the patient's motor threshold but can be higher or lower.

The present invention relating to TMS is equally applicable to the electric stimulation of the brain, such as for example, by deep brain stimulation or transdermal stimulation with electricity such as, for example, the Alpha-Stim cranial electrotherapy stimulation (CES) device. The brain activity of a mammal is modulated by subjecting the mammal to electric stimulation with variable electric pulse intervals for a time sufficient to modulate said brain activity wherein an improvement in a physiological condition or a clinical condition is achieved. Preferably, the electric stimulation is in a random fashion.

In another embodiment of the present invention, the randomized variable pulse intervals are spaced in a time period according to a Gaussian distribution having a determined peak and defined cutoffs at both ends of the contiguous time period. This embodiment can be utilized to treat specific brain wave frequencies/areas associated with a specific brain dysfunction disease or physical conditions described herein.

### Brief Description of the Drawings

Fig. 1 shows a patient being treated with an rTMS device.
Fig. 2 shows a patient being treated with an electric stimulation device.
Fig. 3 shows the variability of a TLL sequence in an electric or magnetic pulses over a six (6) second treatment period.
Fig 4 shows a Gaussian distribution probability graph.

### Detailed Description of the Invention

For the purposes of the present invention the following definitions are disclosed below. The term "mammal" when used herein includes any mammal but especially humans. Non-human mammals include non-human primates, zoo animals, companion animals (dogs, cats) and performance animals such as race horses and breeding animals. Any reference to "humans" described herein will have applicability to other mammals that exhibit the same physiological or medical conditions. Any reference to "patient" when used herein has applicability to any mammal (preferably humans) that may experience the particular condition to which the patient reference is made. The term "variable pulsed interval" when referring to rTMS means that the magnetic stimulation is delivered over time at different time intervals instead of equal time intervals, i.e., frequency. The various time intervals are completely randomly generated from a software program and the probability of each pulse included in the treatment protocol is weighted by Gaussian distribution with a given mean and standard deviation. Random pulsed intervals also may be based on white noise and/or pink noise pulses. Preferably, pre-programmed white noise generators and pink noise generators are used to determine the random pulsed intervals.

In practicing the present invention, a patient, diagnosed with a physical or medical condition where brain activity is at least partially responsible for the physical or medical condition, is treated with variable TMS pulses between 66.00 milliseconds (ms) and 200.00 ms. A computer program is used to generate the TTL pulses, or other triggers, generated by a TMS apparatus variable pulse intervals and preferably random pulse intervals. Preferably, the TMS is delivered as a repetitive TMS (rTMS). rTMS is administered to the patient with variable pulse intervals for a time sufficient to modulate a brain activity which results in an improvement in the physiological condition or the clinical condition being treated.

In a preferred embodiment, random variable pulse intervals are employed in an rTMS protocol used for a time sufficient to modulate a brain activity resulting in an improvement in a physiological condition or a clinical condition. Preferably, the treatments are administered daily for a week after which the patient's progress will be re-evaluated. The exact timing of the repetitive pulses is not critical. The variability of the pulse intervals can be pre-determined by a healthcare professional by choosing specific pulse intervals of a defined treatment. However, it is preferred to program the rTMS device to deliver random pulse interval of from 66.00 ms to 200.00 ms. A six (6) second pulse train every minute for 30 minutes is preferred resulting in a 30-minute treatment period. The maximum intensity setting of the magnetic pulses is generally limited to the patient's motor threshold or lower but can be administered over the patient's motor threshold. It is preferred to set the peak pulse power/intensity of the rTMS to about 50-90% of the patient's motor threshold. Generally, the power/intensity is from 0.1 - 99% of motor threshold, 10-90% of motor threshold, or 40-85% of the motor threshold.

In a further embodiment of the present invention, the variable pulse intervals are spaced in a contiguous time period according to a Gaussian distribution having a determined peak and defined cutoffs at both ends of the contiguous time period. This embodiment can be utilized to treat specific brain wave frequencies/areas associated with a specific brain dysfunction disease or physical conditions. The peak of the Gaussian distribution and the cutoff end-points on both sides of the peak are not critical to the practice of the present invention and will be based on a variety of factors taken into account by a patient's healthcare professional. In treating a disease or condition associated with alpha brain wave dysfunction a preferred pulse interval peak is 100.00 ms with cut off points at 125.00 ms and 83.00 ms. The variable pulse intervals in each time sequence are randomly generated and included within this time period.

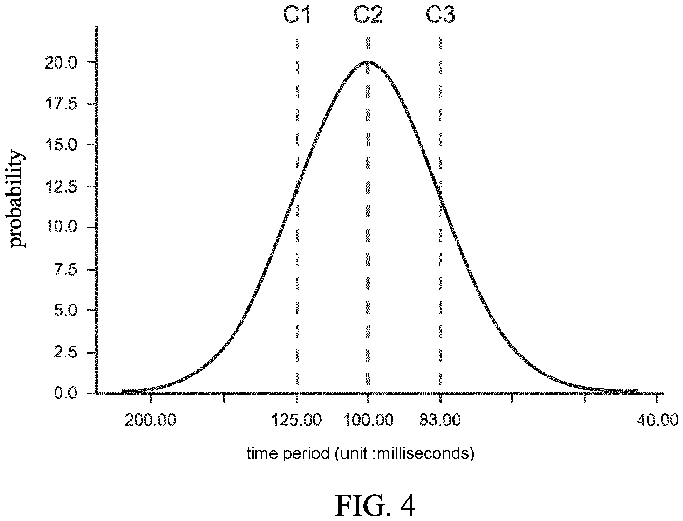

Referring to Fig. 4, a sample Gaussian distribution of period graph is shown where probability (y axis) vs period (x axis) in milliseconds (ms) is shown. The center line C2 represent the peaks of the Gaussian Distribution. Cutoff points C1 and C3 represent the cut off points on both sides of C2. The application of the Gaussian distribution is seen when C1 is 125.00 ms, C2 is 100.00 ms and C3 is 83.00 ms. The range of random pulse intervals would be between 83.00 ms and 125.00 ms. It is worth mentioning that if the X-axis unit displayed on the graph is converted to a reciprocal value regarding to the time period, that is, the time interval is converted to times of appear per second, the distance between C1 and C2 on the X-axis is the same as the distance between C2 and C3 on the X-axis. In other words, the values of the random pulse intervals following Gaussian distribution is times of appear per second.

The rTMS treatments according to the present invention are administered according to well-known protocols employing magnetic coils. The time of actual magnetic stimulation over a set period of time will vary based on each clinical presentation. It is preferred to administer the magnetic stimulation for six continuous seconds per minute of the rTMS session. Sessions can last from 15 to 60 minutes and preferably about 30 minutes. Magnetic coils are placed in close proximity or against a patient's head preferably adjacent to the frontal lobe.

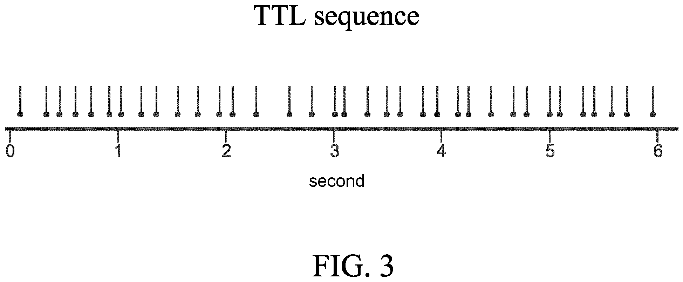

Table. 1 is a chart listing the random magnetic pulse intervals over a 30-minute treatment period. The left-hand column (down) represents the train number, ie, one 6-second treatment per minute. The horizontal rows represent the timing 127 ms from the start, between pulses in milliseconds (ms) in each specific train. i.e, T1, T2, T3, etc. For example, T1 fired at 127 ms after the initial pulse, T2 fired 95 ms after T1, T3 fired 70 ms after T2 and so forth across the Train #1 row. Train #1 lasted 6 seconds. Preferably, pulses in each 6-minute train are newly randomly generated. One minute later Train #2 initiated in the same manner. T11 to T43 are omitted because of space limitations but they are random pulses. Fig. 3 shows the variability of the TLL pulses in a six (6) second treatment period according to the present invention. This is in contrast to a single frequency protocol where all of the pulses would be equidistant to each other across the 6 second treatment segment.

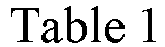

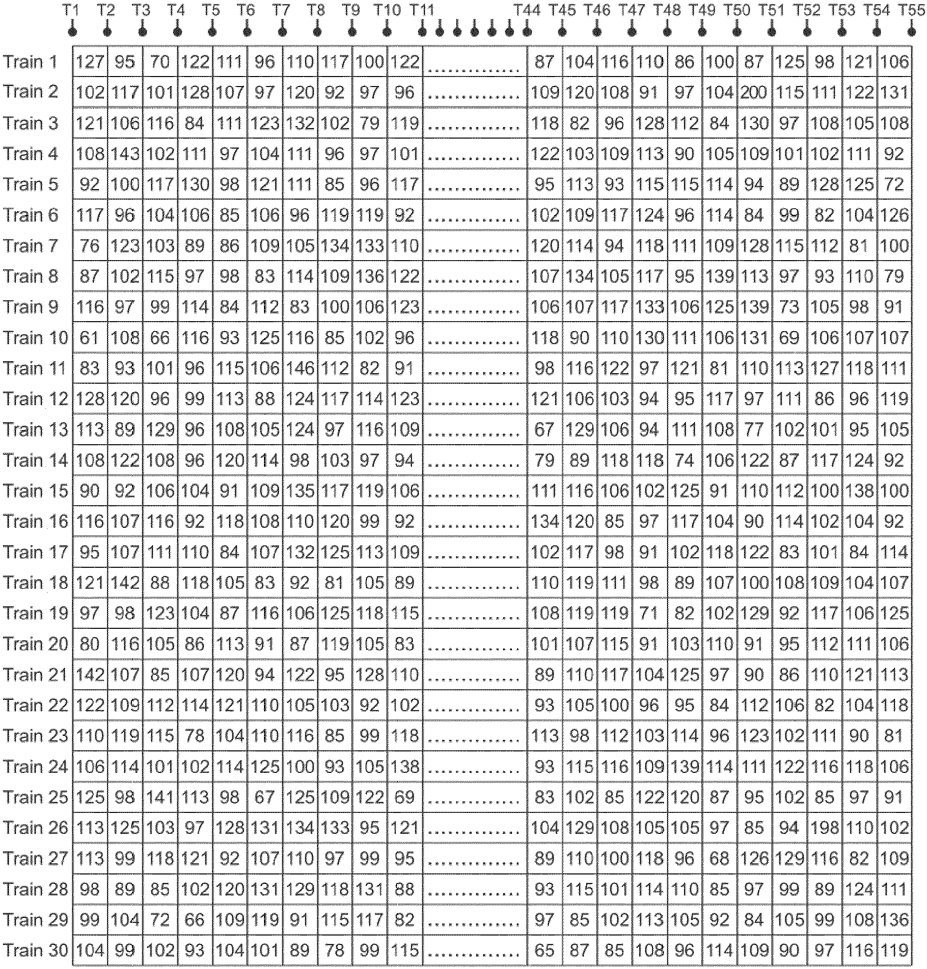

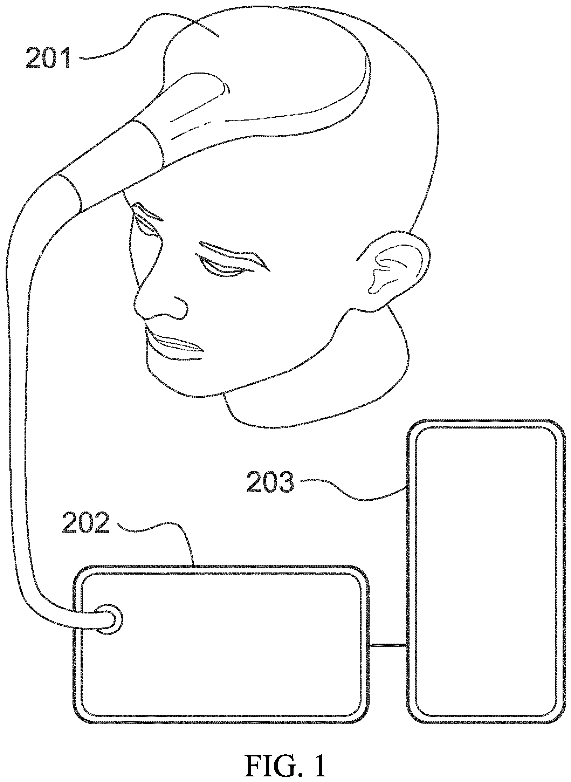

FIG. 1 shows a magnetic stimulation device of the present invention that contains a coil 201, a power source 202 and a computer 203 that controls the stimulation parameters of the variable pulses. The power source 202 and the computer 203 can be manufactured into a single unit control module (not shown). Typically, the control module controls the TTL pulses and the magnetic stimulation delivered by the coil 201. When the power source 202 is turned on an electric current pass through the coiled wires producing a magnetic field. The computer 203 is programed to direct the coil 201 to produce variable pulses according to the present invention. In administering the magnetic stimulation treatment to a patient, the encased magnet coil component is positioned on or in close proximity to the head receiving the treatment.

An electric stimulation apparatus includes two or more electrode pads that adhere to the scalp, a power source configured to produce an electric current in said electrodes and a computer program that directs the electric stimulation of the variable pulses according to the present invention as described herein. In a preferred embodiment of an electric stimulation apparatus, the power source of the electric stimulation apparatus is a battery which provides ease of use in medical treatments including an over-the-counter at-home transcutaneous electric nerve stimulation (TENS unit) device. In administering the electric stimulation treatment to a patient, the electrode pads are positioned contiguous or adjacent to the body part receiving the treatment.

In another embodiment of the present invention, deep brain stimulation(DBS) is administered using random electric pulses to control a variety of brain dysfunction disorders. In deep brain stimulation electrodes are implanted into the region of the brain that is dysfunctional. The electrodes deliver the electric pulse to the target region of the brain and are connected via wires to a pulse generator or neurostimulator. The pulse generator or neurostimulator provides the electric current to the electrodes to deliver the random electric pulse to the targeted brain region. The pulse generator or neurostimulator is programmed to deliver random electric pulses when needed. The pulse generator/neurostimulator is typically surgically implanted under the skin. The random electric pulses are not derived from the patient's EEG data or data derived from a patient's EEG data

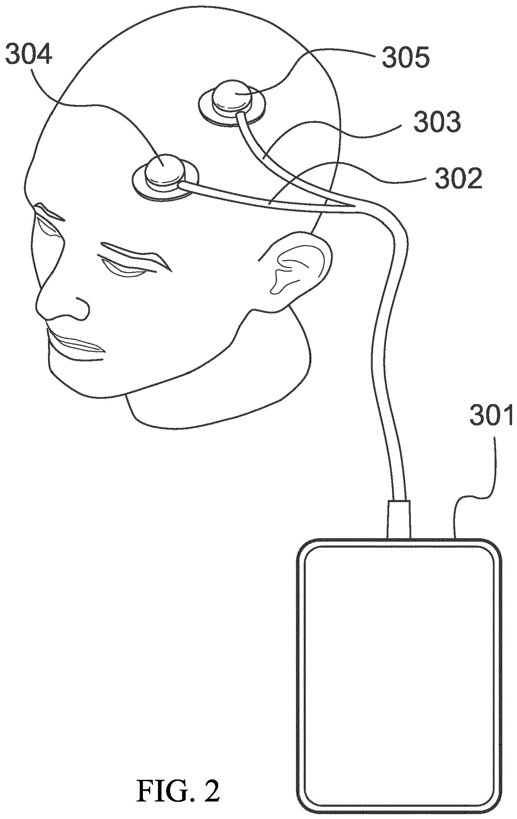

FIG. 2 shows a battery powered electric stimulation device of the present invention that contains an electric pulse generator 301 powered by a battery (not shown), a pair of electrode pads 304 and 305 connected to the control module 301 with wires 302 and 303. The control module is grounded by ground wire 306. A digital or computer program in the control module directs the variable electric stimulation according to the present invention.

The following examples illustrate the practice of the present invention but should not be construed as limiting the scope of the claims. No EEG data or data derived from EEG data were used in programming the variable/random pulse intervals used in the examples

### Example 1: Treating PTSD (Post Traumatic Stress Disorder) and Substance Use Disorder (SUD)

A 25-year-old male military veteran patient, diagnosed with PTSD and SUD, is treated with random variable repetitive transcranial magnetic stimulation (rTMS) according to the present invention for four (4) days. Prior to the rTMS treatment, the patient's motor threshold is determined. The patient receives 6 seconds of random magnetic stimulation every 60 seconds for 30 minutes at a power of 80% of the patient's motor threshold. At a subsequent follow-up examination with the patient reports improvement in night time sleep, mood, tinnitus, muscle pain, stopped drinking alcohol, stopped smoking marijuana and attention span following 4 days of this daily treatment.

### Example 2: Treating Long COVID

A 56-year-old female patient was diagnosed with long COVID and couldn't smell for over one year. She was treated with random variable repetitive transcranial magnetic stimulation (rTMS) according to the present invention for two (2) weeks. Prior to the rTMS treatment, the patient's motor threshold was determined. The patient received 6 seconds of random magnetic stimulation every 60 seconds for 30 minutes at a power of 80% of the patient's motor threshold. Immediately after the first treatment her sense of smell returned. At a subsequent follow-up examination with the patient, she reports improvement in night time sleep, fatigue, muscle pain and shortness of breath.

### Example 3: Treating Alzheimer's Disease

A 72-year-old woman patient, diagnosed with moderate Alzheimer's disease, is treated with random variable repetitive transcranial magnetic stimulation (rTMS) according to the present invention for two (2) weeks for a total of 10 rTMS treatments. Prior to the rTMS treatment, the patient's motor threshold is determined. The patient receives 6 seconds of random magnetic stimulation every 60 seconds for 30 minutes at a power of 75% of the patient's motor threshold. At a subsequent follow-up examination with the woman patient reports improvement in night time sleep, memory, problems with talking and reading, problems processing new situations and attention span following 4 days of this daily treatment.

### Example 4: Treating Autism Spectrum Disorder

A non-verbal 16-year-old male patient, diagnosed with Autism Spectrum Disorder (ASD), is treated with random variable repetitive transcranial magnetic stimulation (rTMS) according to the present invention for two (2) days. Prior to the rTMS treatment, the patient's motor threshold is determined. The patient receives 6 seconds of random magnetic stimulation every 60 seconds for 30 minutes at a power of 80% of the patient's motor threshold. On the third day after two rTMS treatment, the patient starts verbalizing single words. After 2 weeks of treatments that patient is talking in sentences.

### Example 5: Improving Physical Performance

A 54-year-old male Baha 1000 race driver desired to improve his stamina in order to be more competitive in long distance motor racing is treated with random variable repetitive transcranial magnetic stimulation (rTMS) according to the present invention for 5 weeks. Prior to the rTMS treatment, the race driver's motor threshold is determined. The runner received 6 seconds of random magnetic stimulation every 60 seconds for 30 minutes at a power of 70% of the patient's motor threshold. Six (6) weeks after the start of treatment the concentration, endurance and focus improved markedly based on his performance in the Baha 1000 race.

### Example 6: Improving Attention Deficit Disorder (ADD) and Anxiety

A 60-year-old male patient with chronic Attention Deficit Disorder (ADD) and anxiety is treated with random variable repetitive transcranial magnetic stimulation (rTMS) according to the present invention. Prior to the rTMS treatment, the patient's motor threshold is determined. The patient receives 6 seconds of random magnetic every 60 seconds for 30 minutes at a power of 80% of the patient's motor threshold. At a subsequent follow-up examination with the patient, he reports improvement in night time sleep, mood, and attention span following 4 days of this daily treatment.

### Example 7: Reversing Paralysis from the Waste Down

A 41-year-old male experienced deep sea scuba diver involved in a dive greater than 150 feet below the surface of the ocean resurfaced too quickly that resulted in paralysis from his waist down for a period of 7.5 years. The paralyzed male was treated with random variable repetitive transcranial magnetic stimulation (rTMS) according to the present invention for 6 weeks (Mon-Fri). Prior to the rTMS treatment, the paralyzed diver's motor threshold was determined. The paralyzed diver received 6 seconds of random magnetic stimulation every 60 seconds for 30 minutes at a power of 70% of the patient's motor threshold. Within 2 weeks after the start of treatment the diver was able to feel his legs and move his toes. At the end of five (5) weeks the diver was able to stand up.

### Example 8: Decreasing Seizures and Improving Cognition, Coordination and Muscle Control

An 8-year-old female patient has not been able to walk, talk or use her left arm since the age of three (3) and had 70 to 100or more daily uncontrollable grand mal seizures. Prior to treatment with random pulse rTMS the girl was experiencing 70 - 100 or more seizures per day. The girl was treated with random variable repetitive transcranial magnetic stimulation (rTMS) according to the present invention for 3 weeks (Mon-Fri). Prior to the rTMS treatment, the girl's motor threshold was determined. The girl received 6 seconds of random magnetic stimulation every 60 seconds for 30 minutes at a power of 85% of the girl's motor threshold. Within 3 weeks after the start of treatment (15 sessions) the girl was able to walk, talk and could grab things with her left hand. The girl's number of daily seizures was reduced from 70 - 100 to less than 20 seizures/day.

### Example 9: Treating an Alzheimer's Patient With rTMS According to a Gaussian Distribution

An 83-year-old male Alzheimer's patient, diagnosed with moderate to severe Alzheimer's disease, is treated with random variable repetitive transcranial magnetic stimulation (rTMS) by administering a total of 10 rTMS treatments over a two-week period where the pulse intervals were between 83 ms and 125 ms. The pulses were administered as a Gaussian distribution pattern having a peak at 100 ms and cutoffs at 83ms and 125 ms. Prior to the rTMS treatment, the patient's motor threshold is determined. The patient receives 6 seconds of random magnetic stimulation every 60 seconds for 30 minutes at a power of 80% of the patient's motor threshold. At a subsequent follow-up examination with the male patient reports improvement in night time sleep, memory, problems with talking and reading, and attention span following 2 days of this daily treatment.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A programable rTMS apparatus which comprises a magnetic coil to deliver rTMS pulses at variable pulse intervals , characrterized in that: the variable pulse intervals are random variable pulse intervals determined by Gaussian distribution, white noise or pink noise.

2. The programable rTMS apparatus of Claim 1, further comprises a computer program (203) to direct the coil (201) to deliver rTMS pulses at variable pulse intervals .

3. The programable rTMS apparatus of Claim 1, wherein the variable pulse intervals are determined by white noise and/or pink noise.

4. The programable rTMS apparatus of Claim 1, wherein the variable pulse intervals are spaced in a contiguous time period according to a Gaussian distribution having a determined peak and defined cutoffs at both ends of the contiguous time period.

5. The programable rTMS apparatus of Claim 4, wherein the determined peak is a pulse of 100.00 ms, a first cutoff at a pulse of 125.00 ms and a second cutoff at a pulse of 83.00 ms.

6. The programable rTMS apparatus of Claim 1, wherein the variable pulse intervals are between 66.00 milliseconds ms and 200.00 ms.

7. An rTMS apparatus and a programmable system comprises a magnetic coil to deliver rTMS pulses at variable pulse intervals , characrterized in that: the variable pulse intervals are random variable pulse intervals
determined by Gaussian distribution, white noise or pink noise

8. An electric stimulation apparatus which comprises:
a. two or more electrode pads,
b. a power source configured to produce an electric current in said electrode pads and
c. a digital program that directs the electric stimulation of variable pulses,
characrterized in that:
the variable pulses are delivered at variable pulse intervals determined by by Gaussian distribution , white noise or pink noise .

9. .The electric stimulation apparatus of Claim 8 wherein the power source is a battery.

10. The electric stimulation apparatus of Claim 8, which is a transcutaneous electrical nerve stimulation device (TENS Unit) or deep brain stimulator (DBS).

11. The apparatus of any one of Claims 8 to 10, wherein the variable pulse intervals are determined by white noise and pink noise.

12. The apparatus of any one of Claims 8 to 10, wherein the variable pulse intervals are spaced in a contiguous time period according to a Gaussian distribution having a determined peak and defined cutoffs at both ends of the contiguous time period.

13. The apparatus of Claim 12, wherein the determined peak is a pulse of 100.00 ms, a first cutoff at a pulse of 125.00 ms and a second cutoff at a pulse of 83.00 ms.

14. The apparatus of any one of Claims 8 to 10, wherein the variable pulse intervals are between 66.00 milliseconds (ms) and 200.00 ms.
